# EUROPÄISCHE PATENTSCHRIFT

(11) **EP 1 545 560 B1**
(45) Veröffentlichungstag und Bekanntmachung des Hinweises auf die Patenterteilung: **28.07.2010**
(21) Anmeldenummer: 03770876.5
(22) Anmeldetag: 23.09.2003
(51) Int. Cl.: A61K 31/7088, A61K 38/45, A61K 39/395, A61K 31/235, A61K 31/355, A61K 38/13, A61P 5/48

(54) **ZUSAMMENSETZUNGEN ZUR HEMMUNG DER PROTEINKINASE C ALPHA ZUR BEHANDLUNG VON DIABETES MELLITUS**
COMPOSITIONS FOR INHIBITING PROTEIN KINASE C ALPHA FOR THE TREATMENT OF DIABETES MELLITUS
COMPOSITIONS POUR L'INHIBITION DE LA PROTEINE KINASE C ALPHA POUR LE TRAITEMENT DU DIABETE SUCRE

(30) Priorität: 24.09.2002 DE 10244453
(43) Veröffentlichungstag der Anmeldung: 29.06.2005
(73) Patentinhaber: Phenos GmbH, 30625 Hannover (DE)
(72) Erfinder: MENNE, Jan, c/o Phenomiques GmbH, 30625 Hannover (DE); HALLER, Hermann, Medizinische Hochschule Hannover, 30625 Hannover (DE)
(74) Vertreter: von Kreisler Selting Werner
(86) Internationale Anmeldenummer: PCT/DE2003/003165
(87) Internationale Veröffentlichungsnummer: WO 2004/028516

(56) Entgegenhaltungen:
- DE-A- 19 740 384
- US-A- 5 674 902
- US-A- 6 063 814
- MENNE J ET AL: "Protein kinase C alpha deficiency ameliorates the development of diabetic nephropathy in mice with streptozotocin-induced hyperglycemia" KIDNEY AND BLOOD PRESSURE RESEARCH, Bd. 24, Nr. 4-6, 2001, Seite 231, XP008029527 Joint Scientific Meeting of the Nephrology Society and the German Working Group for Clinical Nephrol;Munster, Germany; September 29-October 02, 2001 ISSN: 1420-4096
- MENNE JAN ET AL: "Proteinkinase C alpha deficiency ameliorates the development of diabetic nephropathy in mice with streptozotocin-induced hyperglycemia" JOURNAL OF THE AMERICAN SOCIETY OF NEPHROLOGY, Bd. 12, Nr. Program and Abstract Issue, September 2001 (2001-09), Seite 151A, XP008029528 ASN (American Society of Nephrology)/ISN (International Society of Nephrology) World Congress of Nep;San Francisco, CA, USA; October 10-17, 2001 ISSN: 1046-6673
- MEIER MATTHIAS ET AL: "Knockout of protein kinase C alpha protects against the development of albuminuria but not renal hypertrophy." DIABETES, Bd. 52, Nr. Supplement 1, 2003, Seite A50, XP008029526 63rd Scientific Sessions of the American Diabetes Association;New Orleans, LA, USA; June 13-17, 2003 ISSN: 0012-1797 (ISSN print)
- KANG NINGLING ET AL: "Differential expression of protein kinase C isoforms in streptozotocin-induced diabetic rats" KIDNEY INTERNATIONAL, Bd. 56, Nr. 5, November 1999 (1999-11), Seiten 1737-1750, XP002278386 ISSN: 0085-2538
- DATABASE BIOSIS [Online] BIOSCIENCES INFORMATION SERVICE, PHILADELPHIA, PA, US; 2000, CARMO ANALIA ET AL: "Effect of cyclosporin-A on the blood-retinal barrier permeability in streptozotocin-induced diabetes" XP002278387 Database accession no. PREV200100092751 & MEDIATORS OF INFLAMMATION, Bd. 9, Nr. 5, 2000, Seiten 243-248, ISSN: 0962-9351
- DATABASE BIOSIS [Online] BIOSCIENCES INFORMATION SERVICE, PHILADELPHIA, PA, US; Juni 1998 (1998-06), KOYA DAISUKE ET AL: "Protein kinase C activation and the development of diabetic complications" XP002292425 Database accession no. PREV199800300736 & DIABETES, Bd. 47, Nr. 6, Juni 1998 (1998-06), Seiten 859-866, ISSN: 0012-1797
- DATABASE BIOSIS [Online] BIOSCIENCES INFORMATION SERVICE, PHILADELPHIA, PA, US; 23. Oktober 2001 (2001-10-23), VENUGOPAL SENTHIL KUMAR ET AL: "alpha-Tocopherol inhibits hyperglycemia-induced superoxide anion release from human monocytes via inhibition of PKC-alpha" XP008034017 Database accession no. PREV200200276157
- DATABASE MEDLINE [Online] US NATIONAL LIBRARY OF MEDICINE (NLM), BETHESDA, MD, US; September 1997 (1997-09), HEMPEL A ET AL: "High glucose concentrations increase endothelial cell permeability via activation of protein kinase C alpha." XP002292426 Database accession no. NLM9285638 & CIRCULATION RESEARCH. SEP 1997, Bd. 81, Nr. 3, September 1997 (1997-09), Seiten 363-371, ISSN: 0009-7330
- DATABASE MEDLINE [Online] US NATIONAL LIBRARY OF MEDICINE (NLM), BETHESDA, MD, US; Juni 1996 (1996-06), BUSUTTIL S J ET AL: "Antisense suppresssion of protein kinase C-alpha and -delta in vascular smooth muscle." XP002292427 Database accession no. NLM8661186 in der Anmeldung erwähnt & THE JOURNAL OF SURGICAL RESEARCH. JUN 1996, Bd. 63, Nr. 1, Juni 1996 (1996-06), Seiten 137-142, ISSN: 0022-4804
- DATABASE BIOSIS [Online] BIOSCIENCES INFORMATION SERVICE, PHILADELPHIA, PA, US; September 2001 (2001-09), TAKAHASHI TOMOYA ET AL: "Cyclosporin A promotes hair epithelial cell proliferation and modulates protein kinase C expression and translocation in hair epithelial cells" XP002292428 Database accession no. PREV200100483758 in der Anmeldung erwähnt & JOURNAL OF INVESTIGATIVE DERMATOLOGY, Bd. 117, Nr. 3, September 2001 (2001-09), Seiten 605-611, ISSN: 0022-202X

## Beschreibung

Die vorliegende Erfindung betrifft die Verwendung von Inhibitoren der Aktivität der Proteinkinase C-α (PKC-α) zur Herstellung einer pharmazeutischen Zusammensetzung zur Behandlung und/oder Prävention von diabetischer Retinopathie, diabetischer Neuropathie und diabetischer Nephropathie.

Diabetes mellitus ist einer der häufigsten Erkrankungen in der westlichen Welt, die ungefähr 5 % der Bevölkerung betrifft. Diabetes mellitus wird in einen Diabetes Typ I, welcher in der Regel bereits während der Jugend auftritt, und den auch als Alterszucker bezeichneten Diabetes Typ II unterteilt. Durch eine Störung des Glucosestoffwechsels kommt es bei beiden Diabetes-Formen zu dauerhaft erhöhten Glucosewerten im Blut, was bei den betroffenen Patienten nach mehreren Jahren zu unterschiedlichen Komplikationen führt. Die häufigsten und gleichzeitig am meisten gefürchteten Komplikationen sind die diabetische Retinonopathie, welche zur Erblindung führt, die diabetische Neuropathie, welche Fuß- oder Beinamputation nach sich ziehen kann, und die diabetische Nephropathie.

Eine diabetische Nephropathie entwickelt sich bei ungefähr 40 % aller Diabetes-Patienten und ist weltweit die häufigste Ursache für ein chronisches Nierenversagen und eine Dialysebehandlung. Etwa 30 bis 40 % aller neuen Dialysepatienten weisen eine diabetische Nephropathie auf. Da sich die diabetische Nierenschädigung nur langsam entwickelt, ist es von großer klinischer Bedeutung, bereits frühzeitig Patienten zu identifizieren, die ein erhöhtes Risiko tragen, eine Niereninsuffizienz zu entwickeln, um geeignete therapeutische Schritte einzuleiten. Eines der ersten klinischen Zeichen einer beginnenden Nierenschädigung ist das Auftreten einer sogenannten Mikroalbuminurie. Dabei kommt es zu einer Ausscheidung von 30-300 mg Albumin im 24-Stunden-Sammelurin. Im Normalfall werden weniger als 30 mg Albumin pro Tag ausgeschieden. Eine Mikroalbuminurie tritt unter den gegenwärtigen therapeutischen Bedingungen bei etwa 25 % der Diabetiker mit Diabetes Typ I oder Typ II auf (Alzaid, Diabetes Care, 19 (1996), 79-89; Klein et al., Diabetes Care, 22 (1999), 743-751; Valmadrid et al., Arch. Intern. Med., 160 (2000), 1093-1100). Das Risiko, dass sich eine Niereninsuffizienz entwickelt, ist bei Patienten mit Mikroalbuminurie etwa 10-mal höher als bei Patienten mit normaler Albuminausscheidung. Eine diabetische Nephropathie, welche durch eine Proteinurie von mehr als 300 mg/Tag und/oder eine eingeschränkte Nierenfunktion gekennzeichnet ist, entwickelt sich bei etwa 5 bis 10 % aller Patienten mit Diabetes und Mikroalbuminurie pro Jahr. Auch das Risiko, dass sich eine diabetische Retinopathie entwickelt, ist bei Diabetikern mit Mikroalbuminurie im Vergleich zu Diabetikern ohne Mikroalbuminurie erheblich gesteigert (Vigstrup und Mogensen, Acta Ophthalmol. (Copenh), 63 (1985), 530-534).

Wie Langzeitstudien mit mehr als zehnjährigem Follow-up zeigten, ist die kardiovaskuläre Mortalität bei Typ II- und Typ I-Diabetiker bereits im Stadium der Mikroalbuminurie im Vergleich zu Diabetikern ohne Mikroalbuminurie etwa zweifach erhöht, auch nach Adjustierung für die konventionellen Risikofaktoren wie Cholesterin und Hypertonie (Rossing et al., Bmj, 313 (1996), 779-784; Gerstein et al., Diabetes Care, 23 (2000), Suppl. 2: B35-39; Valmadrid et al., 2000). Eine gesteigerte kardiovaskuläre Mortalität ist auch bei Patienten mit Mikroalbuminurie ohne Diabetes mellitus nachzuweisen (Gerstein et al., Jama, 286 (2001), 421-426).

Es gibt verschiedene Hypothesen, warum die Mikroalbuminurie ein äußerst wichtiger Marker für die Entstehung von Komplikationen bei Patienten mit Diabetes ist. Gemäß der sogenannte "Steno-Hypothese" (Deckert, Feldt-Rasmussen et al., Diabetologia, 32 (1989), 219-226) ist der Verlust von negativ, also anionisch, geladenen Molekülen in der extrazellulären Matrix verantwortlich für die Entstehung von Albuminurie, diabetischer Retinopathie und kardiovaskulärer Komplikationen, beispielsweise koronarer Herzerkrankung. Diese Hypothese stützt sich auf sowohl am Menschen als auch an tierischen Modellsystemen erhobenen Daten und ist in den letzten Jahren durch Ergebnisse weiterer Arbeitsgruppen bestätigt worden.

In der Niere wird der Urin in den Nierenkörperchen, den sogenannten Glomeruli, abgepresst. Um das Durchtreten von Eiweißen, beispielsweise Albumin, zu verhindern, ist die Blutseite von der Urinseite durch eine als Basalmembran bezeichnete Membran getrennt. Die Basalmembran weist kleine Poren auf, die kleineren Molekülen den Durchtritt durch die Basalmembran ermöglichen, während Eiweißmoleküle aufgrund ihrer Größe die Membran nicht passieren können. Bei Patienten mit Mikroalbuminurie kommt es dennoch zum Durchtritt kleiner Eiweiße wie Albumin, obwohl die Porengröße zunächst nicht zunimmt. Als Erklärung für dieses Phänomen konnte gezeigt werden, dass in den Poren beziehungsweise am Porenrand Moleküle negativer Ladung vorhanden sind, welche die ebenfalls negativ geladenen Eiweiße abstoßen (Kverneland, Feldt-Rasmussen et al., Diabetologia, 29 (1986), 634-639; Deckert, Feldt-Rasmussen et al., Kidney Int., 33 (1988), 100-106; Kverneland, Welinder et al., Diabetologia, 31 (1988), 708-710). Bei diesen Molekülen mit negativen Ladungen handelt es sich um Proteoglycane. Proteoglycane sind komplexe Makromoleküle, die aus Proteinen bestehen, an die kovalent Polysacchard-Ketten angelagert sind. Die Polysaccharid-Ketten bestehen überwiegend aus Heparansulfat und sind stark negativ geladen. Das im Körper am häufigsten vorkommende Proteoglycan ist Perlecan. Perlecan ist ein 460 kd großes Protein und hat mehrere Polysaccharid-Seitenketten (Murdoch und Iozzo, Virchows Arch. A. Pathol. Anat. Histopathol., 423 (1993), 237-242; Iozzo, Cohen et al., Biochem. J., 302 (1994), 625-639; Murdoch, Liu et al., J. Histochem. Cytochem., 42 (1994), 239-249). Bei Patienten mit Diabetes und Mikroalbuminurie ist Heparansulfat in der glomerulären Basalmembran kaum vorhanden. Auch bei Patienten mit fortgeschrittener diabetischer Nephropathie lässt sich Heparansulfat nicht mehr in der Basalmembran nachweisen, obwohl die Proteinketten noch vorhanden sind. Dieser Effekt wird durch eine verminderte Heparansulfatsynthese unter hyperglykämischen Bedingungen, wie sie bei Diabetikern vorkommen, erklärt (Parthasarathy und Spiro, Diabetes, 31 (1982), 738-741; Deckert, Feldt-Rasmussen et al., 1988; Nakamura und Myers, Diabetes, 37 (1988), 1202-1211; Nerlich und Schleicher, Am. J. Pathol., 139 (1991), 889-899; Makino, Ikeda et al., Nephron, 61 (1992), 415-421; Scandling und Myers, Kidney Int., 41 (1992), 840-846; Vernier, Steffes et al., Kidney Int., 41 (1992), 1070-1080; Tamsma, van den Born et al., Diabetologia, 37 (1994), 313-320; Iozzo und San Antoniom, J. Clin. Invest., 108 (2001), 349-355). Des weiteren konnte gezeigt werden, dass Heparansulfat-Proteoglycane nicht nur die glomeruläre Filtration von Albumin durch die negative Ladung verhindern, sondern wahrscheinlich auch für die Integrität der Porengröße innerhalb der Basalmembran mitverantwortlich sind (Deckert, Kofoed-Enevoldsen et al., Diabetologia, 36 (1993), 244-251). So führt der Verlust von Heperansulfat-Proteoglycanen mit Fortschreiten der Niereninsuffizienz zu einer Zerstörung der Mikrostruktur der Basalmembran. Diese Veränderungen könnten erklären, weshalb es im Verlauf der diabetischen Nephropathie zu einer großen, nicht-selektiven Proteinurie mit Verlust von größeren Proteinen wie Immunglubolin kommt. Heparansulfat-Proteoglykane sind auch starke Inhibitoren der mesangialen Expansion im Nierenkörperchen. Dies ist von großem Interesse, da es bei Diabetes-Patienten klassischerweise zu einer Expansion des mesangialen Bindegewebes kommt. Es ist daher nicht überraschend, dass der Heparansulfat-Proteoglykan-Verlust bei Diabetes-Patienten als eine wichtige Ursache für die mesangiale Expansion angeschuldigt wird.

Der Verlust von Heparansulfat tritt bei Diabetikern jedoch nicht nur in der Niere auf, sondern in fast allen anderen Organen. So kommt es zu einer deutlichen Verminderung von Heparansulfat im Bindegewebe der Retina, des Skelettmuskels, der Arterienwände und der Haut sowie auf roten Blutkörperchen. Auch Endothelzellen weisen eine verminderte Synthese von Heparansulfat auf (Yokoyama, Hoyer, et al., Diabetes, 46 (1997), 1875-1880; van der Pijl, Daha et al., Diabetologia, 41 (1998), 791-798). Da Heperansulfat-Proteoglykane wichtige antithrombotische Eigenschaften aufweisen, kann der Verlust von Heperansulfat-Proteoglykanen zu der Entstehung von Mikrothromben, beispielsweise in den Netzhautgefäßen, beitragen und somit die Entstehung einer diabetischen Retinopathie begünstigen (Marcum, Fritze et al., Am. J. Physial., 245 (1983), H275-33; Marcum, McKenney et al., J. Clin. Invest., 74 (1984), 341-350; Marcum und Rosenberg, Biochemistry, 23 (1984), 1730-1737; Marcum, Atha et al., J. Biol. Chem., 261 (1986), 7507-7517). Weitere wichtige antiatherosklerotische Funktionen von Heperansulfat-Proteoglykanen (HSPG) bestehen darin, dass HSPG die Proliferation von glatten Gefäßmuskelzellen hemmen, wodurch es zur Entstehung von arteriellen Gefäßläsionen kommt. HSPG hemmen ferner die Bindung von Monozyten (Entzündungszellen) an das subendotheliale Bindegewebe. HSPG hemmen auch die subendotheliale Bindung und Ablagerung von Lipoprotein a und oxidierten LDL, welche eine entscheidende Rolle bei der Entstehung von Arteriosklerose haben. HSPG sind auch wichtige Regulatoren bei der Angiogenese, das heißt bei der Ausbildung von neuen Gefäßen in geschädigten Körperbereichen (Rosenberg, Shworak et al., J. Clin. Invest., 100 (1997), S67-75; Pillarisetti, Trens Cardiovasc. Med., 10 (2000), 60-65; Iozzo und San Antonio, 2001). Der Verlust von Heparansulfat-Proteoglykan ist daher nicht nur bei der Entstehung der diabetischen Nephropathie und der diabetischen Retinopathie von Bedeutung, sondern auch bei der Entstehung kardiovaskulärer Komplikationen.

Ein weiterer Aspekt ist, dass es bei Patienten mit Hypertonie zu einer Mikroalbuminurie kommt. Bislang wurde dieses Phänomen über einen erhöhten Druck in den Nierenkörperchen erklärt, wobei angenommen wurde, dass vermehrt Albumin "abgequetscht" wird. Wenn dies jedoch zutrifft, muss davon ausgegangen werden, dass Patienten mit gleichbleibend hohem arteriellen Blutdruck ebenfalls ein hohes kardiovaskuläres Risiko haben, unabhängig davon, ob sie eine Mikroalbuminurie aufweisen. Dies trifft jedoch nicht zu, wie in mehreren prospektiven Untersuchungen gezeigt werden konnte. Hypertensive Patienten mit Mikroalbuminurie haben eine etwa zweifach höhere kardiovaskuläre Morbidität und Mortalität als ähnlich hypertensive Patienten mit ansonsten vergleichbarem Risikoprofil, beispielsweise Hypercholesterinämie, Raucheranamnese und Diabetes (Sleight, J. Renin Angiotensin Aldosterone Syst., 1 (2000), 18-20; Crippa, J. Hum. Hypertens., 16 (Suppl. 1) (2002), S74-7; Diercks, van Boven et al., Can. J. Cardiol., 18 (2002), 525-535). Dementsprechend ist die Mikroalbuminurie ein unabhängiger Risikoparameter für die Entstehung und Prognose von kardiovaskulären Erkrankungen. Dies lässt sich nur damit erklären, dass es bei Patienten mit Mikroalbuminurie zu einer Veränderung im gesamten Gefäßsystem kommt. Bislang ist jedoch unklar, welche Störung bei Patienten mit Hypertonie der Mikroalbuminurie zugrunde liegt.

Das der vorliegenden Erfindung zugrundeliegende technische Problem besteht darin, Mittel bereitzustellen, die zur Therapie von Mikroalbuminurie bei Patienten mit Diabetes mellitus, eingesetzt werden können, um die mit Diabetes assoziierten Spätschäden diabetische Retinopathie, diabetische Nephropathie und diabetische Neuropathie, zu behandeln und/oder diesen vorzubeugen.

Die vorliegende Erfindung löst das ihr zugrundeliegende technische Problem durch die Verwendung gemäß Anspruch 1.

Im Stand der Technik wurde bislang angenommen, dass die β2-Isoform der Proteinkinase C für die Entstehung der diabetischen Komplikationen verantwortlich ist. Zum einen wird die β2-Isoform im Gewebe diabetischer Tiere vermehrt produziert (Inoguchi et al., Proc. Natl. Acad. Sci. USA, 89 (1992), 11059-11063) und zum anderen führt der Proteinkinase C-β-spezifische Inhibitor LY333531 bei Nagetieren mit Typ I- und Typ II-Diabetes zu einer geringeren Proteinurie als Zeichen einer verminderten Nierenschädigung (Ishii et al., J. Mol. Med., 76 (1998), 21-31; Koya et al., Faseb J., 14 (2000), 439-447.

Erfindungsgemäß erzeugte Proteinkinase C-α-"knock out"-Mäuse, die keine Proteinkinase C-α bilden können, entwickelten überraschenderweise nach Induktion eines Diabetes mittels Streptozotozin keine Albuminurie. Hingegen entwickelten Kontrolltiere, die die bis auf die Veränderung der Proteinkinase C-α-Expression genetisch weitgehend identisch waren, eine deutliche Albuminurie. Die weitere Untersuchung der "knock out"-Tiere ergab erfindungsgemäß vollkommen überraschend, dass die Tiere unter diabetischen Bedingungen in der Lage waren, Heparansulfat in normaler Konzentration zu bilden. Hingegen konnten die Kontrolltiere unter diabetischen Bedingungen kaum noch Heparansulfat bilden.

Erfindungsgemäß durchgeführte histologische Untersuchungen ergaben weitere signifikante Veränderungen bei den Proteinkinase C-α-"knock out"-Mäusen. Unter Verwendung immunhistochemischer Verfahren konnte erfindungsgemäß gezeigt werden, dass das Fehlen der Proteinkinase c-α weitere gravierende Unterschiede in der Expression von VEGF (vascular endothelial growth factor) und des dazugehörigen Rezeptors (VEGF-R II) nach sich zieht. Während bei diabetischen Kontrolltieren ein signifikanter Anstieg der exprimierten Mengen von VEGF und des VEGF R II-Rezeptors nachgewiesen werden konnte, wurde bei den Proteinkinase C-α-"knock out"-Tieren ein deutlich geringerer Anstieg der exprimierten Mengen von VEGF und des VEGF R II-Pezeptors festgestellt. Dieses Ergebnis ist sofern von immenser Bedeutung, als eine gesteigerte VEGF-Expression als einer der wichtigsten Mediatoren für die Entstehung einer diabetischen Retinopathie angesehen wird (Aiello und Wong, Kidney Int. Suppl., 77 (2000), S113-9; Benjamin, Am. J. Pathol., 158 (2001), 1181-1184).

Aus den erfindungsgemäßen Ergebnissen ergibt sich, dass der Proteinkinase C-α bei der Regulation der Heparansulfat-Proteoglykan-Bildung und bei der Ausprägung einer Proteinurie eine zentrale Rolle zukommt. Die erfindungsgemäßen Ergebnisse zeigen auch, dass der Proteinkinase C-α bei der Manifestation der Proteinurie eine erheblich wichtigere Rolle als der Proteinkinase C-β zukommt, wobei die Proteinkinase C-β allerdings offensichtlich in der Lage ist, zumindest einen Teil der Funktionen der Proteinkinase C-α zu übernehmen. Die erfindungsgemäßen Ergebnisse zeigen ferner, dass eine Hemmung der Proteinkinase C-α-Isoform sowohl gezielt Schutz vor der Entwicklung der diabetischen Spätfolgen diabetische Nephropathie, diabetische Retinopathie sowie diabetische Neuropathie.

Offenbart wird auch zur Behandlung und/oder Prävention von Gefäß-Krankheiten, Herz-Kreislauf-Krankheiten, mit Proteinurie assoziierten Nierenerkrankungen, diabetischen Spätschäden und/oder kardiovaskulären Komplikationen bei Patienten mit Diabetes mellitus, kardiovaskulären Komplikationen bei Patienten mit Hypertonic und/oder kardiovaskulären Komplikationen bei Patienten mit Hypercholesterinämie Agenzien zu verwenden, die die Expression und/oder Aktivität der Proteinkinase C-α (PKC-α) vermindern oder hemmen.

Im Zusammenhang mit der vorliegenden Erfindung werden unter "Krankheiten" oder "Erkrankungen" Störungen der Lebensvorgänge in Organen oder im gesamten Organismus mit der Folge von subjektiv empfundenen beziehungsweise objektiv feststellbaren körperlichen, seelischen beziehungsweise geistigen Veränderungen verstanden. Unter "Komplikationen" oder "Spätschäden" werden Folgeerkrankungen oder Sekundärkrankheiten verstanden, das heißt eine zu einem primären Krankheitsbild hinzutretende zweite Erkrankung.

Es wird offenbart, dass es sich bei den zu behandelnden Krankheiten insbesondere um Gefäß-Krankheiten, Herz-Kreislauf-Krankheiten, mit Proteinurie assoziierte Nierenerkrankungen, Diabetes mellitus mit und ohne assoziierte Spätschäden und/oder kardiovaskuläre Komplikationen, Hypertonie mit und ohne assoziierte kardiovaskuläre Komplikationen und/oder Hypercholesterinämie mit und ohne assoziierte kardiovaskuläre Komplikationen, handelt.

Im Zusammenhang mit der vorliegenden Offenbarung werden unter "Gefäß-Krankheiten" oder "Gefäß-Erkrankungen" insbesondere Krankheiten der Arterien verstanden, die zu funktionellen oder organischen Durchblutungsstörungen führen. In bevorzugter Ausführungsform der Erfindung ist die Gefäß-Krankheit eine periphere arterielle Verschlusskrankheit. Unter einer "arteriellen Verschlusskrankheit" wird eine Erkrankung verstanden, die durch stenosierende beziehungsweise obliterierende Veränderungen an den Arterien verursacht werden und zu Durchblutungsstörungen mit Ischämie in versorgungsabhängigen Geweben oder Organen führen. Bei Diabetes mellitus kommt es insbesondere zu chronischen Verschlusskrankheiten, die unter anderem durch obliterierende Arteriosklerose, auch Angiopathien und Angioneuropathien, verursacht werden.

Unter "Herz-Kreislaufkrankheiten" werden Krankheiten und Störungen verstanden, die die Funktion von Herz und Kreislauf, beispielsweise den Füllungszustand und Tonus des Kreislaufsystems, die Auswurfleistung des Herzens, die neuralen und humoralen Kopplungsmechanismen zwischen Herz und Kreislauf etc. betreffen. Insbesondere handelt es sich bei den Herz-Kreislauf-Erkrankungen um koronare Herzerkrankung, Herzinfarkt und Schlaganfall.

Unter "koronarer Herzerkrankung" wird die klinische Manifestation einer primären Koronarinsuffizienz verstanden, wobei es durch Einengung oder Verschluss von Herzkranzgefäßen zu einer Verminderung der Durchblutung und damit der Zufuhr von energieliefernden Substraten und Sauerstoff zum Herzmuskel kommt.

Unter einem "Herzinfarkt" wird die Nekrose eines umschriebenen Herzmuskelbezirks, die meistens akut als Komplikation bei chronischer koronarer Herzkrankheit auftritt, verstanden. Die Ursache für einen Herzinfarkt sind anhaltende kritische Mangeldurchblutung bei Koronarinsuffizienz und länger andauernden Koronargefäßspasmen insbesondere im Bereich einer vorbestehenden exzentrischen Koronarstenose. Der Herzinfarkt manifestiert sich häufig bei körperlicher oder psychischer Belastung infolge der Steigerung des Sauerstoffbedarfs des Herzmuskels oder bei akuter Unterbrechung der Blutversorgung.

Unter "Schlaganfall" oder "Apoplexie" wird ein ichämischer Hirninfarkt infolge artieller Durchblutungsstörung des Gehirns verstanden. Der Schlaganfall wird durch Embolien verursacht, die von arteriosklerotischen Veränderungen extrakranieller Gefäße oder vom Herzen ausgehen, seltener infolge einer Stenose oder zerebraler Mikroangiopathien.

Unter "mit Proteinurie assoziierten Nierenerkrankungen werden insbesondere parenchymatöse Nierenerkrankungen verstanden, die durch das Vorhandensein von Eiweißen im Harn charakterisiert sind. Bei der Proteinurie kann es sich um eine glomeruläre Proteinurie, eine tubuläre Proteinurie oder eine glomerulär-tubuläre Mischproteinurie handeln. Die ausschließlich renale Ausscheidung von Albumin und Transferrin charakterisiert die selektive Proteinurie, die beispielsweise bei Minimal-change-Nephropathie auftritt. Bei der nicht-selektiven Proteinurie ist auch IgG im Harn nachweisbar. Diese Proteinurie-Form ist beispielsweise bei Nierenamyloidose zu finden, aber auch im fortgeschrittenen Stadium der diabetischen Nephropathie. Einer tubulären Proteinurie liegen tubolointerstielle Erkrankungen zugrunde, die reabsorptive Prozesse beeinträchtigen, was die Exkretion niedermolekularer Proteine zur Folge hat. Klinisch ist die tubuläre Proteinurie insbesondere dann bedeutsam, wenn sie mit anderen Defekten des proximalen Tubulus assoziiert ist. Tubuläre Proteinurien kommen unter anderem bei Erkrankungen wie hereditärer Tubuolpathie, renal-tubulärer Azidose, bakteriell oder medikamentös induzierter interstitieller Nephritis, akutem Nierenversagen, Schwermetallvergiftungen, Bence-Jones-Nephropathie und in der postoperativen Phase der Nierentransplantation vor. Der glomerulär-tubulären Mischproteinurie liegen häufig primäre glomeruläre Erkrankungen mit ausgeprägten sekundären interstitiellen Veränderungen zugrunde.

Bei der vorliegenden Offenbarung handelt es sich bei den mit Proteinurie assoziierten Nierenerkrankungen insbesondere um Minimal-change-Nephropathie, sonstige Glomerulopathien, Nierenamyloidose, hereditäre Tubulopathie, renal-tubuläre Azidose, medikamentös oder bakteriell induzierte interstitielle Nephritis, akutes Nierenversagen, Bence-Jones-Nephropathie und die postoperative Phase einer Nierentransplantation.

Im Zusammenhang mit der vorliegenden Erfindung werden unter "Diabetes mellitus" verschiedene Formen der Glycose-Stoffwechselstörung mit unterschiedlicher Ätiologie und Symptomatik verstanden. Gemeinsames Kennzeichen ist ein relativer oder absoluter Mangel an Insulin. Diabetes mellitus-Erkrankungen sind durch eine dauerhafte Erhöhung der Blutglucose (Hyperglykämie) oder durch eine zeitlich inadäquate Verwertung zugeführter Glucose gekennzeichnet. Der Diabetes mellitus wird in Typ I (IDDM) und Typ II (NIDDM) unterteilt.

Diabetes-spezifische und Diabetes-assoziierte chronische Komplikationen sind die Mikroangiopathien Retinopathie, Nephropathie und Neuropathie. Polyneuropathie, diabetischer Fuß, Störung des Skelett-, Stütz- und Bindegewebes sowie Magroangiopathie, insbesondere koronare Herzerkrankung, cerebrale Durchblutungsstörung und periphe arterielle Verschlusskrankheit werden in der Offenbarung berücksichtigt.

Unter "diabetischer Retinopathie" wird eine bei Diabetes mellitus auftretende Mikroangiopathie des Augenhintergrundes verstanden. Formen der diabetischen Retinopathie sind die nicht-proliferative Retinopathie (Hintergrundretinopathie), wie Netzhautblutungen, Mikroaneurysmen, harte Exsudate, Netzhautödem mit Sehschärfenverlust, sowie die proliferative Retinopathie, wobei es zum zusätzlichen Auftreten von Cotton-wool-Herden und Gefäßneubildungen auf und vor der Netzhaut mit Glaskörperblutungen infolge Netzhautischämie durch Gefäßverschlüsse kommt. Die proliferative Retinopathie kann zu Traktionsamotio, neovaskulärem Glaukom und Erblindung führen.

Im Zusammenhang mit der vorliegenden Erfindung wird unter "diabetischer Nephropathie", die auch als diabetische Glomerulosklerose bezeichnet wird, eine Schädigung der glomerulären Nierenkapillaren verstanden. Klinisch äußert sich die diabetische Nephropathie durch Proteinurie, Hypertonie, Ödeme, eine diffuse Verbreiterung der Basalmembran, eine Zunahme des Mesangiums und spätere knötchenartige Verdickungen in den Glomerulusschlingen mit Einengung des Gefäßlumens sowie fibrinoide Ablagerungen in der Kapillarwand und Mikroaneurysmen.

Im Zusammenhang mit der vorliegenden Erfindung wird unter einer "diabetischen Neuropathie" eine Erkrankung der peripheren Nerven verstanden. Insbesondere handelt es sich dabei um die symmetrische distale sensomotorische Polyneuropathie und die autonome Neuropathie. Die periphere Polyneuropathie manifestiert sich typischerweise an den unteren Extremitäten, beginnend an den Füßen, und schreitet nach proximal" weiter und betrifft nicht selten auch die Arme. Die Symptomatologie variiert erheblich, wobei Beschwerden wie Schmerzen, Taubheitsgefühl und Parästhesin häufig zu einer Exazerbation führen.

Unter kardiovaskulären Komplikationen bei Diabetes werden Herz-Kreislauf- und Gefäßerkrankungen, insbesondere periphere Verschlusskrankheit, koronare Herzkrankheit, Herzinfarkt und Schlaganfall verstanden, die infolge von Diabetes mellitus auftreten.

Im Zusammenhang mit der vorliegenden Offenbarung wird unter "Hypertonie" oder "Hypertonus" Bluthochdruck oder Hochdruckkrankheit verstanden, der/die durch eine dauernde Erhöhung des Blutdruckes auf Werte von systolisch von mehr als 140 mmHG und diastolisch von mehr als 90 mmHG gekennzeichnet ist. Unter "mit Hypertonie assoziierten kardiovaskulären Komplikationen" werden Herz-Kreislauf- und Gefäßerkrankungen, insbesondere periphere Verschlusskrankheit, koronare Herzkrankheit, Herzinfarkt und Schlaganfall verstanden, die infolge von Hypertonie auftreten.

Im Zusammenhang mit der vorliegenden Offenbarung wird unter "Hypercholesterinämie" eine erhöhte Cholesterin-Konzentration im Blut verstanden, wobei die Hypercholesterinämie primär oder sekundär infolge von Diabetes auftreten kann. Hypercholesterinämie ist ein Risikofaktor für Arteriosklerose. Unter "mit Hypercholesterinämie assoziierten kardiovaskulären Komplikationen" werden Herz-Kreislauf- und Gefäßerkrankungen, insbesondere periphere Verschlusskrankheit, koronare Herzkrankheit, Herzinfarkt und Schlaganfall verstanden, die infolge von Hypercholesterinämie auftreten.

Im Zusammenhang mit der vorliegenden Erfindung wird unter einer "Proteinkinase C" oder "PKC" eine Proteinfamilie verstanden, die bei der Signalübertragung eine essentielle Rolle spielt, wobei die PKC-Proteine durch Phosphorylierung von Substraten, wie Enzymen, Transkriptionsfaktoren und/oder Cytoskelett-Proteinen intrazellulär regulatorische Funktionen ausüben. Die Aktivierung der PKC-Proteine führt beispielsweise zu einer Aktivierung weiterer Proteinkinasen einschließlich der Mitogen-aktivierten Proteinkinase (MAPK), die somit ein Substrat der PKC-Proteine sind. Proteinkinase C-Proteine sind die Haupt-Phorbolester-Rezeptoren. Die Proteinkinase C-Proteinfamilie umfasst mindestens zwölf Isoformen in Säugerzellen, die in drei unterschiedliche Subfamilien unterteilt werden. Die sogenannten herkömmlichen (conventional) Proteinkinase C-Isoformen (cPKC) umfassen die Isoformen PKC-α, PKC-βI und die Spleißvariante βII sowie PKC-gamma. Die sogenannten neuen Proteinkinase-Isoformen (nPKC) umfassen die Isoformen PKC-delta, PKC-epsilon, PKC-eta und PKC-theta. Die sogenannten αtypischen Proteinkinase C-Isoformen (aPKC) umfassen die Isoformen PKCzeta und PKC-lambda (auch als PKC-iota bekannt). Weitere Isoformen sind PKC-mu (auch als Proteinkinase D bezeichnet) sowie die PKCverwandten Kinasen (PRK), die eventuell eigene Subfammilien darstellen (Toker, Frontiers in Biosciences, 3 (1998), d1134-1147). Die PKC-Isoformen unterscheiden sich sowohl in ihren Aminosäuresequenzen als auch in den die Aminosäuresequenzen codierenden Nucleinsäuresequenzen (Coussens et al., Sciences, 233 (1986), 859-866). Die PKC-Proteine weisen allesamt eine Domänenstruktur auf. Auch ihre zellulären Expressions-muster, die Mechanismen ihrer Aktivierung und ihre Substratspezifität unterscheiden sich.

Die Mehrzahl der Proteinkinase C-Isoformen ist vor Aktivierung nicht membrangebunden und ist im Cytoplasma diffus verteilt. Die Aktivierung der Aktivität jeder Isoform durch Behandlung der Zellen mit der Phorbol-Verbindung 12-O-Tetradecanoylphorbol-13-acetat führt zu Isozymspezifischen Veränderungen der Zellmorphologie sowie zu einer schnellen selektiven Umverteilung der verschiedenen PKC-Isozyme in verschiedene subzelluläre Strukturen. Die Proteinkinase C-α-Isoform reichert sich insbesondere im endoplasmatischen Retikulum und am Zellrand an, während die PKC-βII-Isoform in den actinreichen Mikrofilamenten des Cytoskeletts angereichert wird. Die Substratspezifität der PKC-Isoformen wird zumindest teilweise durch die subzelluläre Verteilung der aktivierten Proteinkinase C-Isozyme vermittelt.

Unter "Proteinkinase C-α" wird ein Protein verstanden, das durch Calciumionen und Dialcylglycerin aktiviert wird, wobei sich die aktivierte Proteinkinase C-α insbesondere im endoplasmatischen Retikulum und am Zellrand anreichert. Die Aminosäuresequenz der PKC-α und die die PKC-α-codierende Nucleinsäuresequenz sind in Coussens et al., Sciences, 233 (1986), 859-866, beschrieben. Das PKC-α-Protein weist eine ähnliche Domänenstruktur wie die übrigen cPKC-Proteine auf. Das Protein umfasst eine Pseudosubstrat-Domäne, eine Cystein-reiche Region, eine Calcium-Bindungsdomäne und eine katalytische Domäne auf. PRC-α kann durch Diacylglycerin, Phorbolester, Phosphatditylserin und Calcium aktiviert werden.

Im Zusammenhang mit der vorliegenden Offenbarung werden unter "Agenzien, die die Expression der Proteinkinase C-α vermindern oder hemmen" solche Mittel verstanden, die sowohl unter in vitro-Bedingungen als auch unter in vivo-Bedingungen die Synthese eines funktionsfähigen PKC-α-Proteins vollständig verhindern oder zumindest reduzieren, wobei die Transkription der die PKC-α-codiereaden DNA-Sequenz in eine komplementäre mRNA-Sequenz, die Prozessierung der mRNA, die Übersetzung der mRNA in eine Polypeptid-Kette, die Prozessierung des Polypeptids und/oder postranslationale Modifikationen des Polypeptids gehemmt oder vermindert werden. Die Verwendung von Agenzien, die die Expression der Proteinkinase C-α vermindern oder hemmen, kann also dazu führen, dass entweder überhaupt kein funktionsfähiges, beispielsweise aktivierbares PKC-α-Protein hergestellt wird oder aber die Menge des erzeugten funktionsfähigen, beispielsweise aktivierbaren PKC-α-Proteins vermindert ist. Die Verwendung von Agenzien, die die Expression der Proteinkinase C-α vermindern oder hemmen, kann aber auch dazu führen, dass ein nicht-funktionsfähiges, beilspielsweise nicht-aktivierbares PKC-α-Protein oder ein nur teilweise funktionsfähiges PKC-α-Protein erzeugt wird.

Im Zusammenhang mit der vorliegenden Offenbarung werden unter "Agenzien, die die Aktivität der Proteinkinase C-α vermindern oder hemmen" solche Mittel verstanden, die sowohl unter in vitro-Bedingungen als auch unter in vivo-Bedingungen die biologische Aktivität des funktionsfähigen PKC-α-Proteins vollständig oder teilweise eliminieren können. Die vollständige oder teilweise Inaktivierung des PKC-α-Proteins kann beispielsweise dadurch erfolgen, dass das verwendete Agens direkt mit dem PKC-α-Protein in Wechselwirkung tritt. Die direkte Interaktion zwischen Agens und PKC-α-Protein kann beispielsweise durch kovalente oder nicht-kovalente Bindung erfolgen. Durch die Wechselwirkung zwischen Agens und PKC-α-Protein kann es beispielsweise auch zu chemischen Änderungen der Proteinkinase kommen, die zu einem Verlust der biologischen Aktivität der Proteinkinase führt. Die Wechselwirkung kann beispielsweise auch zu einem spezifischen Abbau der PKC-α führen. Agenzien, die die Aktivität der Proteinkinase C-α vermindern oder hemmen, können aber auch solche sein, die spezifische Substrate, Zielstrukturen oder Zielmoleküle der PKC-α so modifizieren oder eliminieren oder daran binden, dass auf diese Weise die biologische Aktivität der PKC-α reduziert oder gänzlich unterbunden wird. Agenzien, die die Aktivität der Proteinkinase C-α vermindern oder hemmen, können auch solche sein, die die Translokation der PKC-α nach Aktivierung, beispielsweise durch Phorbol-Behandlung, in das endoplasmatische Retikulum oder an den Rand der Zelle, verhindern, so dass die PKC-α nicht mit ihren spezifischen Substraten, Zielstrukturen oder Zielmolekülen in Wechselwirkung treten kann.

In besonders bevorzugter Ausführungsform der Erfindung handelt es sich bei den erfindungsgemäß, eingesetzten Agenzien um Agenzien, die spezifisch die Expression und/oder Aktivität der PKC-α vermindern oder hemmen, nicht jedoch die Expression und/oder Aktivität anderer PKC-Isoformen, beispielsweise der PKC-β.

Erfindungsgemäß sind die Agenzien, die spezifisch die Expression und/oder Aktivität der PKC-α vermindern oder hemmen Inhibitoren der Proteinkinase C-α-Aktivität. Offenbart werden auch Agenzien, die ausgewählt sind aus der Gruppe bestehend aus Nucleinsäuren, die die Expression des Proteinkinase C-α-Gens vermindern oder hemmen, die Nucleinsäure enthaltenden Vektoren, die Vektoren enthaltenden Wirtszellen, die Expression der Proteinkinase C-α hemmende oder vermindernde Substanzen, die Translokation der Proteinkinase C-α hemmende Substanzen, Antagonisten der Proteinkinase C-α-Aktivität und offenbart wird auch, dass die verwendete Nucleinsäure ausgewählt ist aus der Gruppe bestehend aus
a) einer humane Proteinkinase C-α codierenden Nucleinsäure oder einem Fragment davon,
b) einer Nucleinsäure, die zu der Nucleinsäure nach a) komplementär ist, oder einem Fragment davon,
c) einer Nucleinsäure, die durch Substitution, Addition, Inversion und/oder Deletion einer oder mehrerer Basen einer Nucleinsäure nach a) oder b) erhältlich ist, oder einem Fragment davon; und
d) einer Nucleinsäure, die zu einer Nuclein-säure nach a) bis c) eine Homologie von mehr als 80% aufweist oder einem Fragment davon.

Im Zusammenhang mit der vorliegenden Offenbarung wird unter einer "Proteinkinase C-α oder ein Fragment davon codierenden Nucleinsäure" eine Nucleinsäure verstanden, die ein PKC-α-Protein oder ein Fragment davon codiert, das die funktionellen Domänen, insbesondere die Pseudosubstrat-Domäne, die Cystein-reiche Region, die Calcium-Bindungsdomäne und eine katalytische Domäne der nativen Proteinkinase C-α aufweist. In bevorzugter Ausführungsform der Erfindung codiert die erfindungsgemäß verwendete Nucleinsäure humane PKC-alpha beziehungsweise Teile davon.

"Homologie" bedeutet im Zusammenhang mit der Offenbarung eine Sequenzidentität von mindestens 80 %, vorzugsweise mindestens 85 % und besonders bevorzugt mindestens mehr als 90 %, 95 %, 97 % und 99 %. Der dem Fachmann bekannte Ausdruck der "Homologie" bezeichnet somit den Grad der Verwandtschaft zwischen zwei oder mehreren Nucleinsäuremolekülen, der durch die Übereinstimmung zwischen den Sequenzen bestimmt wird.

Bei der verwendeten Nucleinsäure kann es sich um eine DNA- oder RNA-Sequenz, insbesondere in linearer Form, handeln. Die Nucleinsäure kann eine aus natürlichen Quellen, beispielsweise aus eukaryontischen Geweben, vorzugsweise aus Säugergeweben, bevorzugter aus menschlichen Geweben, isolierte Nucleinsäure sein oder synthetisch hergestellt worden sein.

Es ist insbesondere vorgesehen, dass die als Agens verwendete Nucleinsäure, sofern sie in einem Vektor, insbesondere einem Expressionsvektor, insertiert ist, in einer Wirtszelle in Antisense-Orientierung zu einem Promotor die Expression des Gens der humanen Proteinkinase C-α hemmen kann. Bei Insertion der eingesetzten Nucleinsäure in Antisense-Orientierung in einem Vektor, das heißt bei Vorliegen eines Antisense-Konstrukts der eingesetzten Nucleinsäure, wird die Nucleinsäure als Antisense-Nucleinsäure transkribiert. Bei Transkription des nativen PKC-alpha-Gens der Zelle kann dann das erzeugte Antisense-Transkript der erfindungsgemäß verwendeten Nucleinsäure durch Watson-Crick-Basenpaarungen an das in Sense-Orientierung vorliegende mRNA-Transkript des nativen Proteinkinase C-α-Gens unter Bildung einer Duplex-Struktur binden. Auf diese Weise wird selektiv die Translation der mRNA des nativen PKC-α-Gens in ein Polypeptid unterbunden und die Expression der nativen PKC-alpha spezifisch gehemmt, ohne dass die Expression anderer zellulärer PKC-Isoformen gehemmt wird.

Es wird weiterhin offenbart, dass die zur Erzeugung von Antisense-Konstrukten verwendete Nucleinsäure nicht die gesamte PKC-alpha-codierende Sequenz umfasst, sondern nur Fragmente davon. Diese Fragmente umfassen mindestens 10 Nucleotide, vorzugsweise mindestens 50 Nucleotide, besonders bevorzugt mindestens 200 Nucleotide, wobei die von den Fragmenten überspannten Nucleotid-Bereiche der PKC-alpha-codierenden Sequenz so ausgewählt sind, dass bei Expression der Fragmente in Antisense-Orientierung in einer Zelle eine spezifische Hemmung der Expression der PKC-alpha, insbesondere der humanen PKC-alpha, erfolgt, nicht jedoch eine Hemmung anderer PKC-Isoformen, beispielsweise der PKC-beta-Isoformen.

Es ist vorgesehen, dass die vorstehend genannte Nucleinsäure beziehungsweise ein geeignetes Fragment davon in einem Vektor unter der Kontrolle von mindestens einem Expressions-Regulationselement insertiert ist, wobei die Nucleinsäure oder das Fragment davon in Antisense-Orientierung zu den Expressions-Regulations-elementen insertiert ist. Nach Einführung des Vektors in eine Zelle, beispielsweise eine Säugerzelle, insbesondere eine menschliche Zelle, kann so die Nucleinsäure oder das Fragment davon in Antisense-Orientierung exprimiert und dadurch die Expression der nativen PKC-alpha der Zelle effizient inhibieren. Vorzugsweise handelt es sich bei dem Vektor um ein Plasmid, ein Cosmid, einen Bakteriophagen oder ein Virus.

Offenbart wird daher auch ein Vektor, der eine PKC-alpha-codierende Nucleinsäuresequenz oder ein Fragment unter der funktionellen Kontrolle von mindestens einem Expression-Regulationselement umfasst, wobei die Nucleinsäure oder das Fragment davon in Antisense-Orientierung zu dem Regulationselement insertiert ist. Bei dem Expressions-Regulationselement handelt es sich insbesondere um einen Promotor, eine Ribosomenbindungsstelle, eine Signalsequenz oder einen 3'-Transkriptionsterminator.

Die Offenbarung betrifft eine Wirtszelle, die einen vorstehend beschriebenen Vektor enthält. Bei der Wirtszelle handelt es sich insbesondere um eine Säugerzelle, vorzugsweise eine menschliche Zelle. In besonders bevorzugter Form handelt es sich bei der menschlichen Zelle um eine adulte Stammzelle.

Zur Hemmung der Expression der PKC-alpha werden synthetisch hergestellte Antisense-Oligonucleotide eingesetzt, die mindestens 10 Nucleotide, vorzugsweise mindestens 50 Nucleotide, besonders bevorzugt mindestens 200 Nucleotide umfasst. Solche Antisense-Oligonucleotide können direkt zur Hemmung der PKC-alpha-Expression eingesetzt werden, das heißt müssen nicht in einen Vektor insertiert und unter zellulären Bedingungen exprimiert werden. Dabei handelt es sich bei diesen PKC-α-spezifischen Antisense-Oligonucleotide insbesondere um das Produkt ISIS 3521 von Isis Pharmaceuticals, das ein starker selektiver Inhibitor der Proteinkinase-alpha-Expression ist. In einer weiteren Ausführungsform handelt es sich bei den eingesetzten PKC-a-apezifischen Antisense-Oligonucleotiden um die von Busutti et al., J. Surg. Pathol., 63 (1996), 137-142, beschriebenen Antisense-Oligodesoxynucleotide.

Sowohl die vorstehend genannten Nucleinsäuren, die diese Nucleinsäuren enthaltenden Vektoren als auch die Vektoren enthaltende Wirtszellen können als Agenzien zur Behandlung und/oder Prävention von Gefäß-Krankheiten, Herz-Kreislauf-Krankheiten, mit Proteinurie assoziierten Nierenerkrankungen, mit Diabetes mellitus assoziierten Spätschäden und/oder kardiovaskulären Komplikationen, mit Hypertonie assoziierten kardiovaskuläre Komplikationen und/oder mit Hypercholesterinämie assoziierten kardiovaskulären Komplikationen, beispielsweise im Rahmen einer Gentherapie, eingesetzt werden.

Offenbart wird, dass zur Hemmung oder Verminderung der Expression der Proteinkinase-alpha ein Aktivator der Proteinkinase C-α eingesetzt wird. Vorzugsweise ist der Aktivator eine Phorbol-Verbindung, insbesondere 12-O-Tetradecanoyl-phorbol-13-acetat (TPA) oder Phorbol-12,13-dibutyrat (PDBu). Es ist bekannt, dass eine Inkubation von Zellen mit beispielsweise PDBu über einen Zeitraum von 16 h bis 24 h zu einer vollständigen Down-Regulation der PKC-alpha führt (Busutti et al., J. Surg. Res., 63 (1996), 137-142). Auch ist bekannt, dass eine Behandlung mit einer höheren TPA-Konzentration , beispielsweise 1,6 µM, die PKC-alpha-Expression vollständig hemmt. Daher ist die Behandlung erkrankter Gewebe mit Phorbolestern in einer Konzentration von vorzugsweise mehr als 1,6 µM über einen Zeitraum von mindestens 15 h vorgesehen, um die Expression der PKC-alpha in den betreffenden Geweben oder Organen zu teilweise oder vollständig zu blockieren.

Erfindungsgemäß ist die Verwendung eines Inhibitors zur Hemmung oder Verminderung der Aktivität der Proteinkinase-α vorgesehen. Im Zusammenhang mit der vorliegenden Erfindung wird unter einem "Inhibitor" ein Stoff verstanden, der die biologische Aktivität der Proteinkinase C-α kompetitiv hemmt, die Raumstruktur der PKC-α allosterisch verändert oder die PKC-α durch Substrathemmung inhibiert.

In einer bevorzugten Ausführungsform der Erfindung ist der Inhibitor ein mit der Proteinkinase C-α spezifisch reagierender Antikörper. Unter einem "Antikörper" werden Polypeptide verstanden, die im Wesentlichen durch ein Immunglubolin-Gen beziehungsweise Immunglubolin-Gene codiert werden, oder Fragmente davon, die spezifisch einen Analyten, das heißt ein Antigen, binden und erkennen können. Durch die Bindung des Antikörpers an die PKC-α wird deren biologische Aktivität gehemmt. Erfindungsgemäß können die Antikörper gegen die Proteinkinase C-α als intakte Immungluboline oder als eine Reihe von Fragmenten vorkommen, die durch Spaltung mit verschiedenen Peptidasen erzeugt werden. Der erfindungsgemäß verwendete Begriff "Antikörper" betrifft auch modifizierte Antikörper, zum Beispiel oligomere Antikörper, reduzierte Antikörper, oxidierte Antikörper und markierte Antikörper. Der Begriff "Antikörper" umfasst auch Antikörperfragmente, die entweder durch Modifizierung des gesamten Antikörpers oder de novo unter Verwendung von DNA-Rekombinationsverfahren hergestellt wurden. Der Begriff "Antikörper" umfasst daher sowohl intakte Moleküle als auch Fragmente davon, wie Fab, P(ab')₂ und Fv, die an die epitopischen Determinanten binden können. Diese Antikörperfragmente behalten die Fähigkeit, selektiv an das entsprechende Antigen zu binden. Verfahren zur herstellung von antikörpern beziehungsweise von Fragmenten davon sind im stand der Technik bekannt.

In einer bevorzugten Ausführungsform der Erfindung ist der erfindungsgemäß zur Hemmung der Aktivität der Proteinkinase C-α verwendete Antikörper ein monoclonaler oder polyclonaler Antikörper. Erfindungsgemäß kann der Antikörper auch ein humanisierter Antikörper ist. In einer besonders bevorzugten Ausführungsform der Erfindung sind die zur Hemmung der Aktivität oder Proteinkinase C-α verwendeten Antikörper die von Goodnight et al., J. Biol. Chem., 270 (1995), 9991-10001, beschriebenen.

In einer weiteren bevorzugten Ausführungsform der Erfindung ist vorgesehen, dass der erfindungsgemäß zur Hemmung der PKC-alpha eingesetzte Inhibitor den Phosphorylierungsstatus von Proteinkinase C-α ändert und dadurch die Aktivität der PKC-alpha hemmt oder zumindest vermindert. Aus Tasinato et al., Biochem. J., 334 (1998), 243-249, ist bekannt, dass alpha-Tocopherol die zelluläre Proteinkinase C-alpha inaktivieren kann, indem der Phosphorylierungsstatus von PKC-alpha verändert wird. Daher ist die Verwendung von alpha-Tocopherol zur Hemmung der Aktivität der PKC-alpha und daher zur Behandlung und/oder Prävention von Gefäß-Krankheiten, Herz-Kreislauf-Krankheiten, mit Proteinurie assoziierten Nierenerkrankungen, diabetischen Spätschäden und/oder kardiovaskulären Komplikationen bei Patienten mit Diabetes mellitus, kardiovaskulären Komplikationen bei Patienten mit Hypertonie und/oder kardiovaskulären Komplikationen bei Patienten mit Hypercholesterinämie vorgesehen.

Die Verwendung von Antagonisten der PKC-alpha zur Behandlung und/oder Prävention von Gefäß-Krankheiten, Herz-Kreislauf-Krankheiten, mit Proteinurie assoziierten Nierenerkrankungen, diabetischen Spätschäden und/oder kardiovaskulären Komplikationen bei Patienten mit Diabetes mellitus, kardiovaskulären Komplikationen bei Patienten mit Hypertonie und/oder kardiovaskulären Komplikationen bei Patienten mit Hypercholesterinämie wird offenbart. Im Zusammenhang mit der vorliegenden Offenbarung wird unter einem "Antagonisten" ein Stoff verstanden, der mit der PKCT-alpha um Bindung an ein PKC-alpha-spezifisches Substrat konkurriert, ohne jedoch nach Bindung an das Substrat die gleiche Wirkung wie die PKC-alpha hervorzurufen. Unter Antagonisten werden auch Substanzen verstanden, die aufgrund ihrer Struktur an eine inaktive Konformation eines PKC-alpha-spezifischen Substrates angepasst sind und daher die Aktivierung des Substrates durch die PKC-alpha verhindern.

Als Antagonist zur Hemmung der PKC-alpha-Aktivität wird ein Derivat der PKC-alpha eingesetzt, das zwar an die Substrate der nativen PKC-alpha binden kann, nach Bindung daran allerdings nicht die gleiche biologische Wirkung wie native PKC-alpha entfalten kann.

Im Zusammenhang mit der vorliegenden Offenbarung werden unter "Derivaten" funktionelle Äquivalente oder Abkömmlinge der Proteinkinase C-α verstanden, die unter Beibehaltung der PKC-α-Grundstruktur durch Substitution von Atomen oder Molekülgruppen beziehungsweise -resten erhalten werden und/oder deren Aminosäuresequenzen sich von der natürlicherweise vorkommender menschlicher oder tierischer PKC-α-Molekiile an mindestens einer Position unterscheiden, die aber im wesentlichen einen hohen Grad an Homologie auf der Aminosäureebene aufweisen. Erfindungsgemäß umfasst der Begriff "Derivat" auch Fusionsproteine, bei denen am N-terminalen Teil beziehungsweise am C-terminalen Teil funktionelle Domänen eines anderen Proteins, beispielsweise eines anderen PKC-α-Inhibitors, vorhanden sind.

Die Unterschiede zwischen einem Derivat und nativer PKC-α können beispielsweise durch Mutationen, wie zum Beispiel Deletionen, Substitutionen, Insertionen, Anlagerungen, Basenaustausche und/oder Rekombinationen der die PKC-α-Aminosäuresequenzen codierenden Nucleotidsequenzen entstanden sein. Selbstverständlich kann es sich dabei auch um natürlicherweise auftretende Sequenzvariationen handeln, beispielsweise um Sequenzen aus einem anderen Organismus oder um Sequenzen, die auf natürliche Weise mutiert wurden, oder Mutationen, die mit Hilfe üblicher, auf dem Fachgebiet bekannter Mittel, beispielsweise chemische Agenzien und/oder physikalische Agenzien, gezielt in die entsprechenden Sequenzen eingeführt wurden.

Es wird offenbart, dass als Antagonist zur Hemmung der PKC-alpha-Aktivität ein Analog der PKC-alpha eingesetzt wird. Unter "Analoga" von Proteinkinase C werden Verbindungen verstanden, die keine mit der Proteinkinase C-α identische Aminosäuresequenz aufweisen, deren dreidimensionale Struktur jedoch der der Proteinkinase C-α stark ähnelt. Die offenbarten Analoga der PKC-α weisen vorzugsweise ähnliche Substratspezifitäts-Eigenschaften wie die PKC-α auf, das heißt können an die PKC-alpha-spezifischen Substrate binden, besitzen vorzugsweise jedoch nicht die katalytischen Eigenschaften der PKC-α. Bei den offenbarten Proteinkinase C-α-Analoga kann es sich daher beispielsweise um Verbindungen handeln, die die für die Bindung von Proteinkinase C-α an PKC-α-Substrate verantwortlichen Aminosäurereste in geeigneter Konformation enthalten und die daher die essentiellen Eigenschaften des Bindungsbereiches der Proteinkinase C-α nachahmen können, ohne jedoch die gleichen katalytischen Eigenschaften wie die Proteinkinase C-α zu besitzen.

Es wird weiterhin offenbart dass zur Behandlung und/oder Prävention von Gefäß-Krankheiten, Herz-Kreislauf-Krankheiten, mit Proteinurie assoziierten Nierenerkrankungen, diabetischen Spätschäden und/oder kardiovaskulären Komplikationen bei Patienten mit Diabetes mellitus, kardiovaskulären Komplikationen bei Patienten mit Hypertonie und/oder kardiovaskulären Komplikationen bei Patienten mit Hypercholesterinämie Agenzien eingesetzt werden, die nicht nur die Expression und/oder Aktivität der Proteinkinase C-α (PKC-α) vermindern oder hemmen, sondern gleichzeitig die Expression und/oder Aktivität der Proteinkinase C-β (PKC-β). Takahashi und Kamimura beschreiben in J. Invest. Dermatol., 117 (2001), 605-611, dass das Immunsuppresivum Cyclosporin A die Expression der Proteinkinase-C-Isoformen alpha, beta I und beta II gleichzeitig vermindert. In einer besonders bevorzugten Ausführungsform der Erfindung ist daher die Verwendung von Cyclosporin A zur Verminderung der Expression der PKC-alpha und der PKC-beta und somit zur Behandlung der gemäß Anspruch 1 genannten Krankheiten vorgesehen.

In einer weiteren bevorzugten Ausführungsform der Erfindung ist vorgesehen, dass das Agens, das spezifisch die Expression und/oder Aktivität der Proteinkinase C-α vermindert oder hemmt, in Kombination mit einem Agens verwendet wird, das spezifisch die Expression und/oder Aktivität der Proteinkinase C-β vermindert oder hemmt. Erfindungsgemäß werden unter "Proteinkinase C-β" sowohl die Proteinkinase C-βI als auch die Spleißvariante βII verstanden.

Es wird offenbart, dass das die Expression und/oder Aktivität der Proteinkinase C-β vermindernde oder hemmende Agens ausgewählt ist aus der Gruppe bestehend aus Nucleinsäuren, die die Expression des Proteinkinase C-β-Gens vermindern oder hemmen, die Nucleinsäure enthaltenden Vektoren, die Vektoren enthaltenden Wirtszellen, die Expression der Proteinkinase C-β hemmende oder vermindernde Substanzen, die Translokation der Proteinkinase C-β hemmende. Substanzen, Antagonisten der Proteinkinase C-β-Aktivität und Inhibitoren der Proteinkinase C-β-Aktivität.

Die als Agens einzusetzende Nucleinsaure ist ausgewählt aus der Gruppe bestehend aus
a) einer humane Proteinkinase C-β codierenden Nucleinsäure oder einem Fragment davon,
b) einer Nucleinsäure, die zu der Nucleinsäure nach a) komplementär ist, oder einem Fragment davon,
c) einer Nucleinsäure, die durch Substitution, Addition, Inversion und/oder Deletion einer oder mehrerer Basen einer Nucleinsäure nach a) oder b) erhältlich ist, oder einem Fragment davon; und
d) einer Nucleinsäure, die zu einer Nuclein-säure nach a) bis c) eine Homologie von mehr als 80% aufweist oder einem Fragment davon.

Die verwendete Nucleinsäure codiert humane PKC-alpha beziehungsweise Teile davon.

Bei der verwendeten Nucleinsäure kann es sich um eine DNA- oder RNA-Sequenz, insbesondere in linearer Form, handeln. Die Nucleinsäure kann eine aus natürlichen Quellen, beispielsweise aus eukaryontischen Geweben, vorzugsweise aus Säugergeweben, bevorzugter aus menschlichen Geweben, isolierte Nucleinsäure sein oder synthetisch hergestellt worden sein.

Es wird offenbart, dass die als Agens verwendete Nucleinsäure, sofern sie in einem Vektor, insbesondere einem Expressionsvektor, insertiert ist, in einer Wirtszelle in Antisense-Orientierung zu einem Promotor die Expression des Gens der humanen Proteinkinase C-β hemmen kann. Bei Insertion der eingesetzten Nucleinsäure in Antisense-Orientierung in einem Vektor, das heißt bei Vorliegen eines Antisense-Konstrukts der eingesetzten Nucleinsäure, wird die Nucleinsäure als Antisense-Nucleinsäure transkribiert. Bei Transkription des nativen PKC-β-Gens der Zelle kann dann das erzeugte Antisense-Transkript der verwendeten Nucleinsäure an das in Sense-Orientierung vorliegende mRNA-Transkript des nativen Proteinkinase C-β-Gens unter Bildung einer Duplex-Strulctur binden. Auf diese Weise wird selektiv die Translation der mRNA des nativen PKC-β-Gens in ein Polypeptid unterbunden und die Expression der nativen PYC-β spezifisch gehemmt, ohne dass die Expression anderer zellulärer PKC-Isoformen gehemmt wird.

Es wird weiterhin offenbart, dass die zur Erzeugung von Antisense-Konstrukten verwendete Nucleinsäure nicht die gesamte PKC-β-codierende Sequenz umfasst, sondern nur Fragmente davon. Diese Fragmente umfassen mindestens 10 Nucleotide, vorzugsweise mindestens 50 Nucleotide, besonders bevorzugt mindestens 200 Nucleotide, wobei die von den Fragmenten überspannten Nucleotid-Bereiche der PKC-β-codierenden Sequenz so ausgewählt sind, dass bei Expression der Fragmente in Antisense-Orientierung in einer Zelle eine spezifische Hemmung der Expression der PKC-β, insbesondere der humanen PKC-β, erfolgt, nicht jedoch eine Hemmung anderer PKC-Isoformen.

Es ist vorgesehen, dass die vorstehend genannte Nucleinsäure beziehungsweise ein geeignetes Fragment davon in einem Vektor unter der Kontrolle von mindestens einem Expressions-Regulationselement insertiert ist, wobei die Nucleinsäure oder das Fragment davon in Antisense-Orientierung zu den Expressions-Regulationselementen insertiert ist. Nach Einführung des Vektors in eine Zelle, beispielsweise eine Säugerzelle, insbesondere eine menschliche Zelle, kann so die Nucleinsäure oder das Fragment davon in Antisense-Orientierung exprimiert und dadurch die Expression der nativen PKC-β der Zelle effizient inhibieren. Vorzugsweise handelt es sich bei dem Vektor um ein Plasmid, ein Cosmid, einen Bakteriophagen oder ein Virus.

Die Offenbarung betrifft daher auch einen Vektor, der eine PKC-β-codierende Nucleinsäuresequenz oder ein Fragment unter der funktionellen Kontrolle von mindestens einem Expression-Regulationselement umfasst, wobei die Nucleinsäure oder das Fragment davon in Antisense-Orientierung zu dem Regulationselement insertiert ist. Bei dem Expressions-Regulationselement handelt es sich insbesondere um einen Promotor, eine Ribosomenbindungsstelle, eine Signalsequenz oder einen 3'-Transkriptionsterminator.

Offenbart wird eine Wirtszelle, die einen vorstehend beschriebenen Vektor enthält. Bei der Wirtszelle handelt es sich insbesondere um eine Säugerzelle, vorzugsweise eine menschliche Zelle. In besonders bevorzugter Form handelt es sich bei der menschlichen Zelle um eine adulte Stammzelle.

Zur Hemmung der Expression der PKC-β werden synthetisch hergestellte Antisense-Oligonucleotide eingesetzt, die mindestens 10 Nucleotide, vorzugsweise mindestens 50 Nucleotide, besonders bevorzugt mindestens 200 Nucleotide umfasst. Solche Antisense-Oligonucleotide können direkt zur Hemmung der PKC-β-Expression eingesetzt werden, das heißt müssen nicht in einen Vektor insertiert und unter zellulären Bedingungen exprimiert werden.

Es wird offenbart , zur Hemmung der Aktivität der Proteinkinase C-β einen mit der Proteinkinase C-β spezifisch reagierender Antikörper oder ein geeignetes Fragment davon einzusetzen. Erfindungsgemäß kann es sich bei dem Antikörper um einen monoclonalen oder polyclonalen Antikörper handeln. Der erfindungsgemäß eingesetzte Antikörper kann auch ein humanisierter Antikörper sein.

In einer weiteren bevorzugten Ausführungsform der Erfindung ist vorgesehen, zur Hemmung der Aktivität der Proteinkinase C-β eine Substanz einzusetzen, die den Phosphorylierungsstatus von Proteinkinase C-β ändert.

In weiteren bevorzugten Ausführungsform der Erfindung ist vorgesehen, zur Hemmung der Aktivität der Proteinkinase C-β ein Derivat oder Analog von Proteinkinase C-β einzusetzen, die als Antagonisten der PKC-β wirken. Vorzugsweise handelt es sich bei den erfindungsgemäß eingesetzten Derivaten oder Analoga der PKC-β um Stoffe, die mit der nativen PKC-β um Bindung an PKC-β-spezifische Substrate konkurrieren, ohne jedoch nach Bindung an die Substrate die gleiche Wirkung wie die PKC-β hervorzurufen.

In einer besonders bevorzugten Ausführungsform der Erfindung werden zur spezifischen Hemmung oder Verminderung der Expression und/oder Aktivität der Proteinkinase C-β die in den Dokumenten US 5,491,242, US 5,661,173, US 5,481,003, US 5,668,152, US 5,672,618, WO 95/17182, WO 95/35294 und WO 02/ beschriebenen Verbindungen eingesetzt.

Die Agenzien, die spezifisch die Expression und/oder Aktivität der Proteinkinase C-α (PKC-α) vermindern oder hemmen, können zur Herstellung einer pharmazeutischen Zusammensetzung zur Behandlung und/oder Prävention von koronarer Herzerkrankung, Herzinfarkt, peripherer Verschlusskrankheit, Schlaganfall, mit Proteinurie einhergehenden Nierenerkrankungen, diabetischen Spätschäden und/oder kardiovaskulären Komplikationen bei Patienten mit Diabetes mellitus, kardiovaskulären Komplikationen bei Patienten mit Hypertonus und kardiovaskulären Komplikationen bei Patienten mit Hypercholesterinämie verwendet werden. Dabei handelt es sich bei den kardiovaskulären Komplikationen vorzugsweise um koronare Herzerkrankung, Herzinfarkt, periphere Verschlusskrankheit und Schlaganfall. Bei den diabetischen Spätschäden handelt es sich erfindungsgemäß um diabetische Retinopathie, diabetische Neuropathie und diabetische Nephropathie.

Unter einer "pharmazeutischen Zusammensetzung" oder einem "Arzneimittel" wird ein zu diagnostischen, therapeutischen und/oder prophylaktischen Zwecken verwendetes, also ein die Gesundheit eines menschlichen oder tierischen Körpers förderndes oder wiederherstellendes Gemisch verstanden, das mindestens einen natürlichen oder synthetisch hergestellten Wirkstoff umfasst, der die therapeutische Wirkung hervorruft. Die pharmazeutische Zusammensetzung kann sowohl ein festes als auch ein flüssiges Gemisch sein. Beispielsweise kann eine den Wirkstoff umfassende pharmazeutische Zusammensetzung einen oder mehrere pharmazeutisch verträgliche Komponenten enthalten. Darüber hinaus kann die pharmazeutische Zusammensetzung üblicherweise auf dem Fachgebiet verwendete Zusatzstoffe, beispielsweise Stabilisatoren, Fertigungsmittel, Trennmittel, Sprengmittel, Emulgatoren oder andere üblicherweise zur Herstellung pharmazeutischer Zusammensetzung verwendete Stoffe umfassen.

Offenbart ist insbesondere die Verwendung von Agenzien, die spezifisch die Expression und/oder Aktivität der Proteinkinase C-α (PKC-α) vermindern oder hemmen, als Wirkstoff zur Herstellung eines Arzneimittels zur Therapie und/oder Prophylaxe der vorstehend genannten Krankheiten vorgesehen.

Die zur Herstellung von pharmazeutischen Zusammensetzungen eingesetzten Agenzien sind ausgewählt aus der Gruppe bestehend aus Nucleinsäuren, die die Expression des Proteinkinase C-α-Gens vermindern oder hemmen, die Nucleinsäure enthaltenden Vektoren, die Vektoren enthaltenden Wirtszellen, die Expression der Proteinkinase C-α hemmende oder vermindernde Substanzen, die Translokation der Proteinkinase C-α hemmende Substanzen, Antagonisten der Proteinkinase C-α-Aktivität und Inhibitoren der Proteinkinase C-α-Aktivität.

Besonders bevorzugt handelt es sich bei den zur Herstellung der pharmazeutischen Zusammensetzung verwendeten Agenzien um Antisense-Oligonucleotide des Proteinkinase C-α-codierenden Gens, Tocopherol, Phorbol-Verbindungen, Derivate der Proteinkinase C-α oder Analoga der Proteinkinase C-α.

Die pharmazeutische Zusammensetzung wird zur parenteralen, insbesondere intravenösen, intramuskulären, intrakutanen oder subkutanen Verabreichung verwendet. Vorzugsweise weist das die eingesetzten Agenzien enthaltende Arzneimittel die Form einer Injektion oder Infusion auf.

Die die erfindungsgemäß eingesetzten Agenzien enthaltende pharmazeutische Zusammensetzung kann oral verabreicht werden. Beispielsweise wird das Arzneimittel in einer flüssigen Darreichungsform wie einer Lösung, Suspension oder Emulsion, oder einer festen Darreichungsform wie einer Tablette verabreicht.

Die Offenbarung betrifft daher auch pharmazeutische Zusammensetzungen zur Prävention und/oder Behandlung von koronarer Herzerkrankung, Herzinfarkt, peripherer Verschlusskrankheit, Schlaganfall, mit Proteinurie einhergehenden Nierenerkrankungen, diabetischen Spätschäden und/oder kardiovaskulären Komplikationen bei Patienten mit Diabetes mellitus, kardiovaskulären Komplikationen bei Patienten mit Hypertonus und kardiovaskulären Komplikationen bei Patienten mit Hypercholesterinämie, umfassend mindestens ein Agens, das die Expression und/oder Aktivität der Proteinkinase C-α (PKC-α) spezifisch vermindert oder hemmt, als Wirkstoff.

Die in der pharmazeutischen Zusammensetzung enthaltenen Agenzien sind ausgewählt aus der Gruppe bestehend aus Nucleinsäuren, die die Expression des Proteinkinase C-α-Gens vermindern oder hemmen, die Nucleinsäure enthaltenden Vektoren, die Vektoren enthaltenden Wirtszellen, die Expression der Proteinkinase C-α hemmende oder vermindernde Substanzen, die Translokation der Proteinkinase C-α hemmende Substanzen, Antagonisten der Proteinkinase C-α-Aktivität und Inhibitoren der Proteinkinase C-α-Aktivität.

Die pharmazeutische Zusammensetzung enthält Antisense-Oligonucleotide des Proteinkinase C-αcodierenden Gens, Tocopherol, Phorbol-Verbindungen, Derivate der Proteinkinase C-α oder Analoga der Proteinkinase C-α.

Die erfindungsgemäße pharmazeutische Zusammensetzung enthält insbesondere mindestens einen weiteren Wirkgemäße pharmazeutische Zusammensetzung enthält insbesonderen mindestens einen weiteren Wirkstoff. Bei dem weiteren Wirkstoff handelt es sich insbesondere um ein Agens, das spezifisch die Expression und/oder Aktivität der Proteinkinase C-β vermindert oder hemmt.

Das die Expression und/oder Aktivität der Proteinkinase C-β vermindernde oder hemmende Agens kann ausgewählt sein aus der Gruppe bestehend aus Nucleinsäuren, die die Expression des Proteinkinase C-β-Gens vermindern oder hemmen, die Nucleinsäure enthaltenden Vektoren, die Vektoren enthaltenden Wirtszellen, die Expression der Proteinkinase C-β hemmende oder vermindernde Substanzen, die Translokation der Proteinkinase C-β hemmende Substanzen, Antagonisten der Proteinkinase C-β-Aktivität und Inhibitoren der Proteinkinase C-β-Aktivität.

Die Erfindung wird anhand der folgenden Figuren und Beispiele näher erläutert.

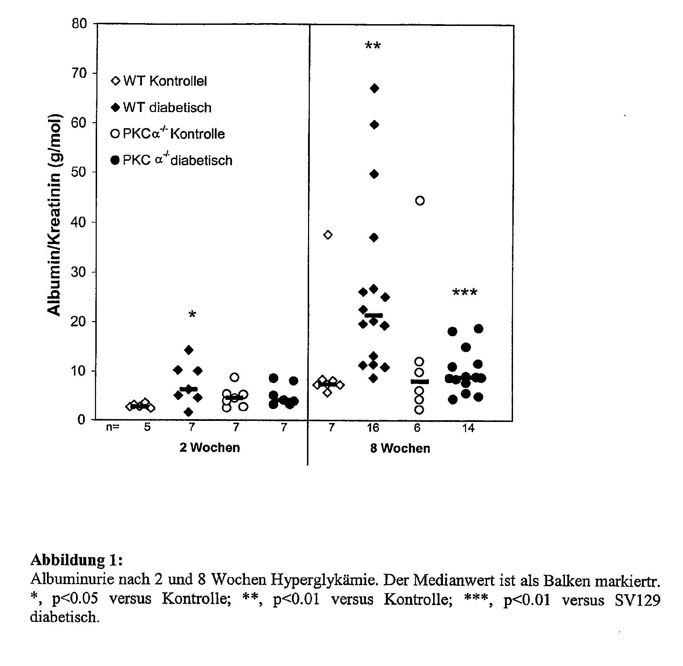

Figur 1 zeigt die Albumin-Ausscheidung im Urin von PKC-alpha-"knock out"-Mäusen mit Diabetes mellitus und ohne Diabetes mellitus (Kontrolle) und SV129-Mäusen mit Diabetes und ohne Diabetes (Kontrolle). Dabei wurde die Albumin-Konzentration unter Verwendung eines indirekten ELISA-Tests bestimmt. Die ermittelten Albumin-Werte wurden auf die Kreatinin-Konzentration bezogen. Die nicht-diabetischen SV129- und PKC-α^{-/-}-Mäuse weisen einen vergleichbaren Albumin/Kreatinin-Quotienten auf, der in der Regel unter 10 g/mol liegt. Hingegen liegt der Quotient bei diabetischen SV129-Mäusen signifikant höher (p = 0,004). Die Werte der diabetischen PKC-α^{-/-}-Mäuse sind signifikant niedriger als die Werte der diabetischen SV129-Mäuse (P = < 0,001). Der Querstrich zeigt den Median an. Die Signifikanz wurde mittels des Mann-Whitney-U-Tests berechnet.

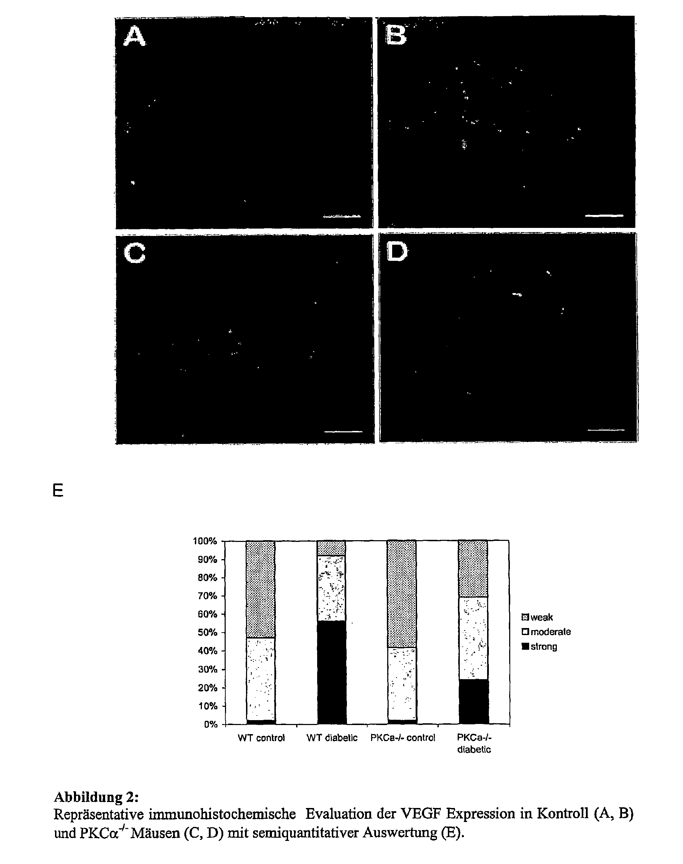

Figur 2 zeigt die glomeruläre VEGF-Expression bei PKC-alpha-"knock out"-Mäusen mit Diabetes mellitus und ohne Diabetes mellitus (Kontrolle) und SV129-Mäusen mit Diabetes und ohne Diabetes (Kontrolle). Dabei wurden pro Tiergruppe jeweils 40 Glomeruli unter Verwendung immunhistochemischer Verfahren semiquantitativ ausgewertet, wobei eine Unterteilung in eine schwache, mittlere und starke Immunfluoreszenz erfolgte. Die Signifikanz wurde mittels des Mann-Whitney-U-Tests berechnet. Die VEGF-Expression ist bei diabetischen Tieren signifikant höher als bei Kontrolltieren (p < 0,001). Jedoch ist die VEGF-Expression bei diabetischen SV129-Tieren signifikant höher als bei diabetischen PKC-alpha^{-/-}-Tieren (p < 0,001).

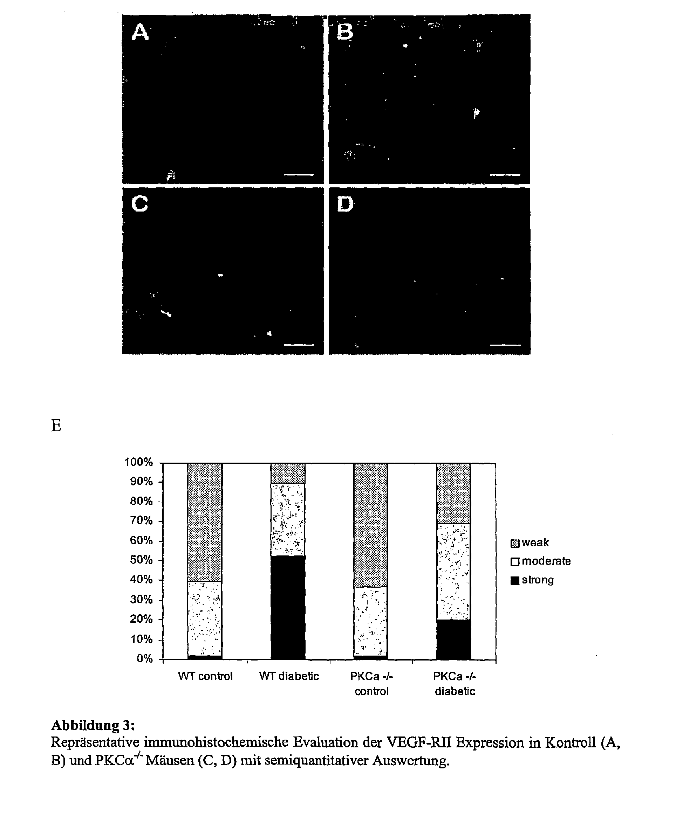

Figur 3 zeigt die glomeruläre VEGF-Rezeptor-II-Expression bei PKC-alpha-"knock out"-Mäusen mit Diabetes mellitus und ohne Diabetes mellitus (Kontrolle) und SV129-Mäusen mit Diabetes und ohne Diabetes (Kontrolle). Dabei wurden pro Tiergruppe jeweils 40 Glomeruli unter Verwendung immunhistochemischer Verfahren semiquantitativ ausgewertet, wobei eine Unterteilung in eine schwache, mittlere und starke Immunfluoreszenz erfolgte. Die Signifikanz wurde mittels des Mann-Whitney-U-Tests berechnet. Die VEGFR-II-Expression ist bei diabetischen Tieren signifikant höher als bei Kontrolltieren (p < 0,001). Jedoch ist die VEGFR-II-Expression bei diabetischen SV129-Tieren signifikant höher als bei diabetischen PKC-alpha^{-/-}-Tieren (p < 0,001).

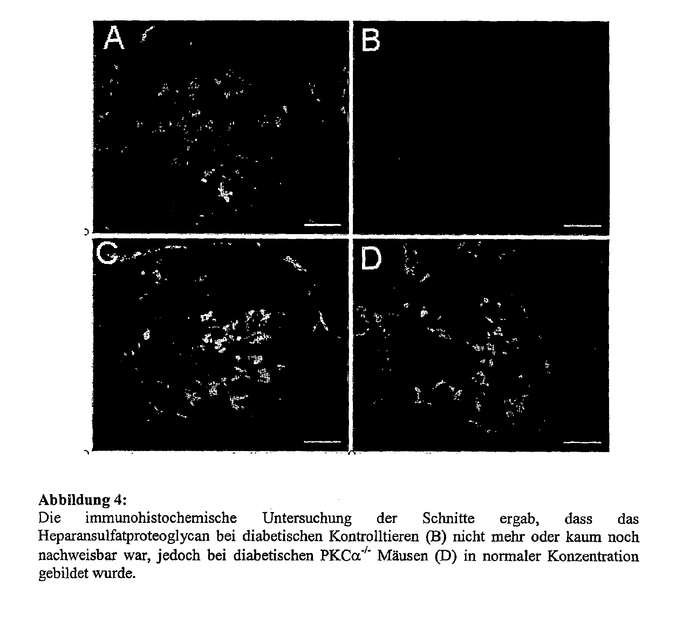

Figur 4 zeigt die glomeruläre Perlecan-Expression bei PKC-alpha-"knock out"-Mäusen mit Diabetes mellitus und ohne Diabetes mellitus (Kontrolle) und SV129-Mäusen mit Diabetes und ohne Diabetes (Kontrolle). Dabei wurden pro Tiergruppe jeweils 40 Glomeruli unter Verwendung immunhistochemischer Verfahren semiquantitativ ausgewertet, wobei eine Unterteilung in eine schwache, mittlere und starke Immunfluoreszenz erfolgte. Die Signifikanz wurde mittels des Mann-Whitney-U-Tests berechnet. Die Perlecan-Expression ist bei diabetischen SV129-Tieren signifikant niedriger als bei SV129-Kontrolltieren (p < 0,001).

### Beispiel 1

### Experimentelle Diabetes-Erzeugung

Bei Mäusen, die unter standardisierten Bedingungen bei 22°C gehalten wurden und freien Zugang zu Futter und Wasser hatten, wurden nach Genehmigung durch die Tierschutzbehörde Niedersachen folgende Versuche durchgeführt.

Vor Beginn des Versuches wurde bei allen Tieren der Blutzuckerspiegel aus dem Serum bestimmt. Die Ergebnisse sind in Tabelle 1 dargestellt. Bei 16 SV129-Kontrollmäusen und 14 SV129-Proteinkinase C alpha-knock out(PKC-α^{-/-})-Mäusen wurde ein Diabetes durch Injektion von Streptozotozin induziert. Streptozotozin führt zu einer Zerstörung der Insulin produzierenden Inselzellen im Pankreas. Durch den resultierenden Insulinmangel kommt es zu dauerhaft erhöhten Blutzuckerspiegeln, d.h. Hyperglykämie, und damit zum Diabetes mellitus. Zur Erzeugung der Hyperglykämie wurden den Tieren an den Tagen 1 und 4 jeweils 125 mg Streptozotozin/kg Körpergewicht intraperitoneal verabreicht. Das Streptozotozin wurde für diese Zwecke in einer 50 mM Na-Citrat Lösung mit einem pH-Wert von 4,5 gelöst. Zur Kontrolle wurde 7 SV129- und 6 PKC-α^{-/-}-Mäusen nur das Lösungsmittel an den Tagen 1 und 4 intraperitoneal verabreicht. Anschließend wurde den Mäusen alle 2 Tage ein Tropfen Blut aus dem Schwanz entnommen, um den Blutzucker zu kontrollieren. Die Blutzuckermessung erfolgte mittels des Glucometer Elite^{®}-Messgeräts von Bayer. Zur Bestimmung wurden Glucometer Elite Sensor^{®} - Teststreifen verwendet.

Am 7-10 Tag waren die Tiere, denen Streptozotozin verabreicht worden war, diabetisch mit Blutzuckerwerten oberhalb von 350 mg/dl. Die Ausgangswerte vor Streptozotozin-Injektion lagen im Schnitt bei 200 mg/dl. Bei Tieren, die nur das Lösungsmittel erhalten hatten, kam es zu keinem Anstieg der Blutzuckerwerte und sie entwickelten keinen Diabetes mellitus. 10 Tage nach der ersten Injektion wurden die Tiere für weitere 8 Wochen beobachtet. Während dieser Zeit wurde der Blutzucker alle zwei Wochen kontrolliert, um sicher zu stellen, dass die diabetischen Tiere weiterhin diabetisch waren. Die Zuckerwerte schwankten bei den diabetischen Tieren während dieser Zeit im Mittel um 500-550 mg/dl und bei den nicht diabetischen Tieren, um 200 mg/dl.

Nach 8 Wochen wurden die Tiere mit dem Narkosemittel Avertin betäubt. In Narkose wurde anschließend aus dem Augenvenenplexus 400 µl Blut und aus der Blase durch Punktion mit einer 27 G Nadel der gesamte Blasenurin entnommen. Anschließend wurden die Nieren mit Ringer-Lactat Lösung über die Bauchaorta perfundiert und die Nieren entfernt. Die Tiere wurden sofort danach in Narkose getötet.Die Blutzuckerwerte wurden anschließend aus dem Serum bestimmt. Die Blutzuckerspiegel finden sich in Tabelle 1 wieder. Wie zu erkennen ist, weisen die diabetischen Tiere etwa 2,5-3-mal höhere Glukose-Werte als zu Beginn des Versuches und auch im Vergleich zu den nicht-diabetischen Kontrolltieren auf.

**Tabelle 1**

| Serumglucose bei diabetischen und nicht-diabetischen Mäusen vor Ver-suchsbeginn und am Versuchsende | | |
|---|---|---|
| | Serumglucose vor Versuchsbe-ginn (mg/dl) | Serumglucose am Versuchsende (mg/dl) |
| SV129 Kontrolle (n = 7) | 205 +/- 40 | 223 +/- 43 |
| SV129 diabetisch (n = 16) | 192 +/- 36 | 505 +/-80* |
| PKC-α^{-/-} Kontrolle (n = 6) | 223 +/- 27 | 197 +/-21 |
| PKC-α^{-/-} diabetisch (n = 14) | 225 +/- 31 | 589 +/- 98* |

| | | |
|---|---|---|
| * p = < 0,001 im Vergleich zu nicht-diabetischen Kontrolltieren | | |

### Beispiel 2

### Bestimmung der Albumin-Konzentration

Die Entstehung einer Albuminurie bei Patienten mit Diabetes ist ein bekanntes Phänomen. Daher wurde die Albuminausscheidung im Urin von PKC-alpha-"knock out"-Mäusen mit Diabetes mellitus und ohne Diabetes mellitus (Kontrolle) und SV129-Mäusen mit Diabetes und ohne Diabetes (Kontrolle) bestimmt. Zu diesem Zweck wurde die Albuminkonzentration im gesammelten Urin bestimmt. Zur Bestimmung der Albuminkonzentration wurde ein indirekter ELISA-Test (Albuwell M^{®}) der Firma Exocell Inc., Philadelphia, USA) verwendet. Dieser ELISA-Test ist für Mausalbumin spezifisch. Die Bestimmung erfolgte entsprechend den Angaben des Herstellers. Um Schwankungen in der Urinausscheidung erfassen zu können, wurden die bestimmten Albuminwerte auf die Kreatininkonzentration im Urin bezogen. Die Ergebnisse sind in Figur 1 dargestellt. Dabei zeigte sich, dass die nicht-diabetischen SV129- und PKC-α^{-/-} -Mäuse einen vergleichbaren Albumin/Kreatinin-Quotienten haben, der in der Regel unter 10 g/mol liegt. Hingegen liegt der Quotient bei diabetischen SV129-Mäusen signifikant höher (p = 0,004). Der Median beträgt 21,5 g/mol versus 7,48 g/mol bei nicht-diabetischen SV129-Kontrolltieren. Im Vergleich dazu kommt es bei diabetischen PKC-α^{-/-}-Mäusen zu keinem signifikanten Anstieg der Albuminurie. Der Albumin/Kreatinin-Quotient liegt immer unter 20 g/mol und der Median findet sich bei 10,2 g/mol. Der Median der nicht-diabetischen PKC-α^{-/-}-Kontrollmäuse liegt bei 8,5 g/mol. Die Werte der diabetischen PKC-α^{-/-}-Mäuse sind signifikant niedriger als die Werte der diabetischen SV129-Mäuse (P = < 0,001). Die Ergebnisse sind in Figur 1 dargestellt.

### Beispiel 3

### Bestimmung der VEGF- und VEGF-Rezeptor-II-Expression

Wie vorstehend in Beispiel 1 ausgeführt, wurden alle Tiere bei Beendigung des Versuches getötet. Unmittelbar danach wurden die Nieren entnommen und bei -70°C eingefroren. Eine weitere Analyse der entnommenen Nieren zeigte in den Nierenkörperchen (Glomeruli) der diabetischen Kontrolltieren eine signifikante Zunahme der Expression des "vascular endothelial growth factor" (VEGF) und des VEGF-Rezeptors II (VEGFR-II). Der Nachweis der VEGF- und VEGFR-II-Expression erfolgte mittels immunhistochemischer Verfahern. Dazu wurden die bei -70°C eingefrorenen Nieren mit einer Dicke von 6 nm kryo-geschnitten und luftgetrocknet. Anschließend wurden die Gefrierschnitte mit kaltem Aceton fixiert, luftgetrocknet und mit Tris-Puffer (TBS: 0.05M Tris-Puffer, 0,15 M NaCl, pH 7.6) gewaschen. Die Gefrierschnitte wurden anschließend 60 Minuten in einer feuchten Kammer mit einem primären polyklonalen "rabbit" Antikörper gegen Maus-VEGF (Santa Cruz, A-20) bzw. VEGFR-II (Santa Cruz, C-1158)inkubiert. Nach erneutem Waschen mit TBS wurden die Schnitte mit einem Cy3 markierten sekundären "anti-rabbit" Antikörper (Jackson Immunresearch Laboratories, 711-165-152) 30 Minuten bei Raumtemperatur inkubiert und nochmals mit TBS gewaschen. Anschließend wurden die Präparate durch ein Zeiss Axioplan-2 Mikroskop (Zeiss, Jena, Deutschland) ausgewertet und photographiert. Bei allen Tieren wurden jeweils 40 Nierenkörperchen ausgewertet, wobei die Fluoreszenz-Intensität in stark, mittel und schwach unterteilt wurde. Dabei fand sich bei diabetischen SV129 Kontrolltieren eine signifikante Zunahme (p <0,001) der VEGF- und VEGF-R-II-Expression gegenüber nicht-diabetischen Kontrolltieren. Im Vergleich dazu war die Zunahme der Expression signifikant weniger ausgeprägt bei diabetischen SV129- und PKC-α^{-/-}-Mäusen (p < 0,001). Die Ergebnisse sind in den Figuren 2 und 3 gezeigt.

### Beispiel 4

### Bestimmung der Perlecan-Expression

Da die festgestellte unterschiedliche VEGF-Expression allein nicht den Unterschied in der Albuminurie erklären konnte, wurde die Expression des Heparansulfat-Proteoglycans Perlecan in den Nieren diabetischer und nicht-diabetischer Tiere unter Verwendung immunhistochemischer Verfahren untersucht. Dabei wurden, wie in Beispiel 3 beschrieben, Kryoschnitte der Nieren angefertigt und eingebettet. Als primärer Antikörper wurde ein monoklonaler Ratten-Antikörper der gegen Maus-Perlecan gerichtet ist verwendet (RDI Systems, A7L6). Als sekundärer Antikörper wurde ein Cy3 markierter "donkey anti-rat" Antikörper (Jackson Immunresearch Laboratories, 712-165-153) verwendet. Die immunhistochemische Untersuchung der Schnitte ergab völlig überraschend, dass Perlecan bei diabetischen Kontrolltieren nicht mehr oder kaum noch nachweisbar war (vgl. Figur 4). So ließ sich weder im Glomerulus noch in der Gefäßwand von Arteriolen Perlecan nachweisen. Hingegen war die Expression von Perlecan bei diabetischen PKC-α^{-/-}-Mäusen unverändert oder nur geringfügig vermindert. Da das Fehlen von Heparansulfat als einer der Hauptmediatoren für die Entstehung einer Proteinurie gilt, ist davon auszugehen, dass dieser Befund das Ausbleiben einer Albuminurie erklären kann.

## Patentansprüche

1. Verwendung von Inhibitoren der Proteinkinase C-α (PKC-α)-Aktivität, zur Herstellung einer pharmazeutischen Zusammensetzung zur Behandlung und/oder Prävention von diabetischer Retinopathie, diabetischer Neuropathie und diabetischer Nephropathie.

2. Verwendung nach Anspruch 1, wobei der Inhibitor der Proteinkinase C-α-Aktivität ein mit der Proteinkinase C-α reagierender Antikörper, insbesondere ein monoklonaler oder polyclonaler Antikörper, wie ein humanisierter Antikörper, ist oder wobei der Inhibitor den Phosphorylierungsstatus von Proteinkinase C-α ändert

3. Verwendung nach einem der Ansprüche 1 oder 2, wobei der Inhibitor der Proteinkinase C-α-Aktivität gleichzeitig ein Inhibitor der Proteinkinase C-β-Aktivität ist.

4. Verwendung nach einem der Ansprüche 1 bis 3, wobei der Inhibitor der Proteinkinase C-α-Aktivität in Kombination mit einem Inhibitor der Proteinkinase C-β-Aktivität eingesetzt wird.

5. Verwendung nach Anspruch 4, wobei der Inhibitor ein mit der Proteinkinase C-β reagierender Antikörper, insbesondere ein monoclonaler oder polyclonaler Antikörper, vorzugsweise ein humanisierter Antikörper, ist.

6. Verwendung nach Anspruch 5, wobei der Inhibitor den Phosphorylierungsstatus von Proteinkinase C-β ändert.

## Claims

1. Use of inhibitors of protein kinase C-α (PKC-α) activity for the preparation of a pharmaceutical composition for the treatment and/or prevention of diabetic retinopathy, diabetic neuropathy and diabetic nephropathy.

2. The use according to claim 1, wherein said inhibitor of protein kinase C-α activity is an antibody reacting with protein kinase C-α, especially a monoclonal or polyclonal antibody, such as a humanized antibody, or wherein said inhibitor alters the status of phosphorylation of protein kinase C-α.

3. The use according to either of claims 1 or 2, wherein said inhibitor of protein kinase C-α activity is at the same time an inhibitor of protein kinase C-β activity.

4. The use according to any of claims 1 to 3, wherein said inhibitor of protein kinase C-α activity is employed in combination with an inhibitor of protein kinase C-β activity.

5. The use according to claim 4, wherein said inhibitor is an antibody reacting with protein kinase C-β, especially a monoclonal or polyclonal antibody, preferably a humanized antibody.

6. The use according to claim 5, wherein said inhibitor alters the status of phosphorylation of protein kinase C-β.

## Revendications

1. Utilisation d'inhibiteurs de l'activité de la protéine-kinase C-α (PKC-α) pour la préparation d'une composition pharmaceutique destinée au traitement et/ou à la prévention de la rétinopathie diabétique, de la neuropathie diabétique et de la néphropathie diabétique.

2. Utilisation selon la revendication 1, dans laquelle l'inhibiteur de l'activité de la protéine-kinase C-α est un anticorps réagissant avec la protéine-kinase C-α, en particulier un anticorps monoclonal ou polyclonal tel qu'un anticorps humanisé, ou dans laquelle l'inhibiteur modifie l'état de phosphorylation de la protéine-kinase C-α.

3. Utilisation selon l'une quelconque des revendications 1 ou 2, dans laquelle l'inhibiteur de l'activité de la protéine-kinase C-α est également un inhibiteur de l'activité de la protéine-kinase C-β.

4. Utilisation selon l'une quelconque des revendications 1 à 3, dans laquelle l'inhibiteur de l'activité de la protéine-kinase C-α est mis en oeuvre conjointement avec un inhibiteur de l'activité de la protéine-kinase C-β.

5. Utilisation selon la revendication 4, dans laquelle l'inhibiteur est un anticorps réagissant avec la protéine-kinase C-β, en particulier un anticorps monoclonal ou polyclonal, de préférence un anticorps humanisé.

6. Utilisation selon la revendication 5, dans laquelle l'inhibiteur modifie l'état de phosphorylation de la protéine-kinase C-β.
